# EUROPEAN PATENT APPLICATION

(11) **EP 4 190 291 A1**
(43) Date of publication of application: **07.06.2023**
(21) Application number: 21212028.1
(22) Date of filing: 02.12.2021
(51) Int. Cl.: A61F 13/20, A61F 13/551, B32B 27/10, B65D 65/38, B65D 65/42, B65D 65/46, D21H 27/10

(54) **WRAPPER FOR ABSORBENT ARTICLE**

(71) Applicant: Ontex BV, 9255 Buggenhout (BE); Ontex Group NV, 9320 Erembodegem (BE)
(72) Inventor: PLATH, Felipe, 02692 Großpostwitz (DE); VANDENBOSSCHE, Jeroen, 9230 Wetteren (BE); LESAGE, Henri, 9900 Eeklo (BE)
(74) Representative: Saurat, Thibault

(57) **Abstract**

The invention pertains to wrapper (22) for a tampon (10), said wrapper comprising a layer consisting essentially of cellulosic fibers (33), a layer of sealing agent (34) arranged onto the layer of cellulosic fibers (33) wherein the layer consisting essentially of cellulosic fibers (33) comprises a basis weight comprised between 15 gsm and 90 gsm. The invention also relates to an assembly comprising a tampon (10) and such wrapper (22).

## Description

### TECHNICAL FIELD

The disclosure pertains to the technical field of absorbent articles for hygiene products and the packaging of such absorbent articles. In particular, the present disclosure relates to individually packaged tampons.

### BACKGROUND

Absorbent articles selected from tampons used to collect vaginal discharges are well known in the art. Typically, these absorbent articles commonly comprise an absorbent material that is rolled and radially-compressed (rolled tampons) or is side-compressed (W-tampon) and are individually enclosed, or packaged, in a wrapper. These wrappers are made out of plastic films such as a polypropylene or polyethylene films since these material have several beneficial physical properties such as flexibility, deformability, hydrophobicity *etc.* as well as for cost reasons. However, these wrappers are always made from petroleum-derived materials and thus are not environment-friendly.

There is still a need to improve the environmental impact of these wrappers and offer a solution that involves less plastic without degrading the performance of the packaging of these absorbent articles.

The invention thereto aims to provide an environment-friendly packaging for tampon.

### SUMMARY OF THE INVENTION

The present invention relates to a wrapper for a tampon, said wrapper comprising a layer consisting essentially of cellulosic fibers and a layer of sealing agent, wherein the layer consisting essentially of cellulosic fibers comprises a basis weight comprised between 15 gsm and 90 gsm. The layer of sealing agent can be arranged on or superposed on, adjacent to or juxtaposed to, the layer of cellulosic fibers.

By using a cellulose pulp such as paper or other natural fibers corresponding to environment friendly materials, the environmental impact of the packaging is lowered. By comprising a basis weight comprised between 15 gsm and 90 gsm, the packaging layer retains the functionality of the plastic film conventionally used. The basis weight is sufficiently high enough to ensure that the wrapper is robust to contain an absorbent article and not tear easily. The basis weight is sufficiently low enough to ensure that the wrapper is flexible and can be easily folded. The basis weight of the layer of cellulosic fibers is preferably measured according to the ISO 536 standard or any standard method.

According to an embodiment, the layer consisting essentially of cellulosic fibers consists essentially of paper material.

According to an embodiment, the wrapper further comprises a layer of hydrophobic material. The layer of hydrophobic material can be arranged on or superposed on, adjacent to or juxtaposed to, the layer of cellulosic fibers.

According to an embodiment, the layer of hydrophobic material comprises a peelable layer that can be separated from the layer consisting essentially of cellulosic fibers.

According to an embodiment, the layer consisting essentially of cellulosic fibers comprise a thickness comprised between 30 µm to 200 µm.

According to an embodiment, the layer consisting essentially of cellulosic fibers comprises cellulosic fibers with an average fiber diameter comprised between 0,5 and 50 µm.

Average fiber length or average fibers diameter can be determined by using any standard image processing analysis method such as measuring said fiber length and/or fiber diameter with a microscope or a similar apparatus and extracting said data (average fiber length, average fiber diameter) from the images.

According to an embodiment, the layer consisting essentially of cellulosic fibers comprises a first sub-layer of cellulosic fibers and a second layer of cellulosic fibers, the cellulosic fibers in the second sub-layer having a greater basis weight than the first sub-layer.

According to an embodiment, the layer of sealing agent has a basis weight comprised between 0.5 gsm and 15 gsm, preferably between 1 gsm and 10 gsm, more preferably between 2 gsm and 5 gsm.

According to an embodiment, the sealing agent is selected from water-based blister varnish, polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid.

According to an embodiment, the wrapper comprises a rectangular configuration, the layer of sealing agent being arranged on the periphery of the wrapper along at least two contiguous edges. For example, the layer of sealing agent is arranged on the periphery of the wrapper along three edges.

According to an embodiment, the layer consisting essentially of cellulosic fibers comprises regenerated cellulosic fibers selected from viscose, acetate and/or rayon.

According to an embodiment, the wrapper comprises a grasping element.

According to an embodiment, the wrapper comprises uncoated portions arranged between an edge of the wrapper and a portion of the wrapper coated by a layer of sealing agent.

According to an embodiment, the wrapper comprises at least one graphic element.

All of these embodiments mentioned above can be taken individually or in combination.

The invention also pertains to an assembly comprising a wrapper as described above and a tampon pledget.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
FIG. 1 illustrates a perspective view of a tampon pledget destined to be individually packaged in a wrapper;
**FIG. 2** illustrates a diagrammatic cross section of a tampon pledget enclosed in a wrapper according to an embodiment of the invention;
**FIG. 3** shows a schematic top view of wrapper in an unfolded configuration according to an embodiment;
**FIG. 4** illustrates a diagrammatic cross section of a tampon pledget enclosed in a wrapper according to an embodiment of the invention;
**FIG. 5** illustrates a top view of a wrapper in a folded configuration according to an embodiment;
**FIG. 6** illustrates a diagrammatic cross section of a tampon pledget enclosed in a wrapper according to an embodiment of the invention;
**FIG. 7** illustrates a rear view of a wrapper in a folded configuration according to an embodiment;
**FIG. 8** shows a schematic top view of wrapper in an unfolded configuration according to an embodiment;
**FIG. 9** shows a schematic top view of wrapper in an unfolded configuration according to an embodiment;
**FIG. 10** illustrates a top view of a wrapper in a folded configuration according to an embodiment;
**FIG. 11** shows a schematic top view of wrapper in an unfolded configuration according to an embodiment.

### DETAILED DESCRIPTION OF THE INVENTION

The present invention concerns the packaging of individually packaged tampons.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints except where otherwise explicitly stated by disclaimer and the like.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.
The term "arranged on a surface" as used herein means that this element is adjacent to, or in contact with, said surface.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The term "adhesive" as used herein is intended to refer to any suitable hot melt, heat sealable material, water or solvent borne adhesive that can be applied, or coated on a surface of the wrapper, or wrapping layer, or paper layer in the required pattern or network of adhesive areas to form the wrapper of the present disclosure. Accordingly, suitable adhesives include conventional hot melt adhesives, pressure-sensitive adhesives and reactive adhesives (i.e., polyurethane).

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene and polyurethane.

As used herein, the term "basis weight" refers to an average weight, or a range of weight, of the component in question, namely the layer consisting essentially of cellulosic fibers or the layer of sealing agent or heat sealable material, per square meter. Terms such as "grammage" or "superficial weight" are equivalent to "basis weight" and is expressed in grams per square meter, g/m² or gsm.

For instance, the basis weight of the layer(s) essentially consisting of cellulosic fibers, *e.g.* paper layer(s), is preferably measured according to the ISO 536 standard or any standard method. The amount of sealing agent or hydrophobic material on the wrapper can be measured by taking a sample of the wrapper where the hydrophobic material or the sealing agent is arranged, weighing said sample before and after removal of the hydrophobic material or sealing agent (in grams) the difference giving the weight of the hydrophobic material or sealing agent, and dividing the weight of hydrophobic material or sealing agent by the area of the sample (in m²) thereby obtaining the amount of peeling agent or sealing agent in gsm.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

As used herein, the terms "cellulosic", "cellulose fibers", "cellulosic fibers" or "cellulosic fibres" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, viscose, rayon, thermomechanical wood pulp, chemical wood pulp, debonded chemical wood pulp, milkweed, regenerated cellulosic fibers or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

The term "graphic" or "graphic element" includes, but is not limited to, any type of design, image, mark, logo, drawing, shape, codes, words, writing, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°. A layer comprising or made out of hydrophobic material will have a lower water/vapor transmission rate that can be measured using standard method such as ASTM F1249 or ASTM E96.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone. The term "layer" used herein also encompasses a coat of, or a coating of, a material.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layer or webs have been formed from many processes such as for example, meltblowing processes, spunbonding processes, and bonded carded web processes.

The term "polymer" or "polymeric material" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "substantially", it is meant at least the majority of the structure or element referred to.

Use of the term "substrate" includes, but is not limited to, woven or nonwoven webs, porous films, ink permeable films, cellulosic fibers, pulp, paper, composite structures, or the like.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "wrapping sheet", "packaging sheet" or "wrapping construction" are equivalent to "wrapper".

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

The term "finished" or "final", when used with reference to a product, means that the product has been suitably manufactured for its intended purpose and can be ready for use.

The term "rayon" refers to a manufactured regenerated cellulose fiber, made from purified cellulose. It has a smooth, soft surface, and is therefore very suitable to be used in a tampon.

As used herein, the terms "material" and "agent" are equivalent and refer to a substance or a mixture of substance with given physical and chemical properties.

Embodiments according to the disclosure will now be described. It is understood that technical features described in one or more embodiments maybe combined with one or more other embodiments without departing from the intention of the disclosure and without generalization therefrom.

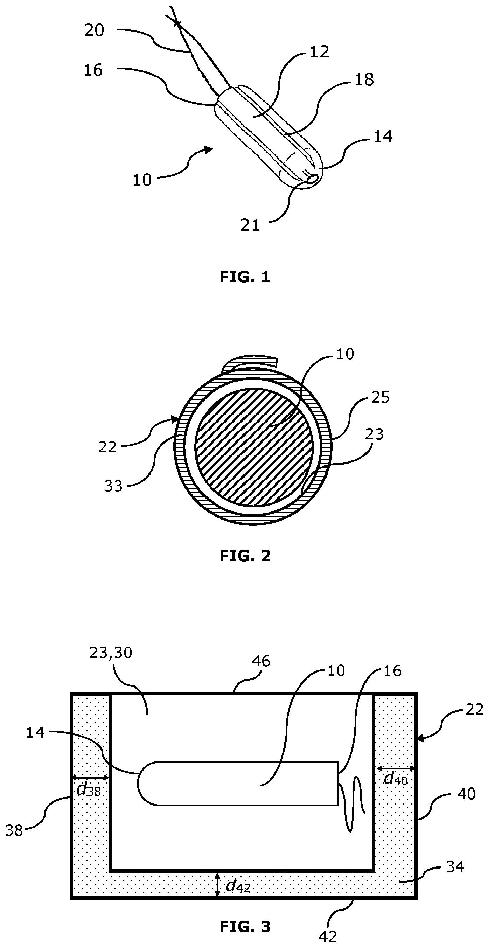

FIG. 1 illustrates a disposable tampon pledget 10 in its finished form. The tampon pledget 10 comprises a cylindrical body 12 extending in a longitudinal direction between an introductory end 14 and a trailing end 16. The tampon pledget 10 comprises an absorbent material such as cotton, viscose, rayon or a combination thereof. Given the objective of the present invention to provide an environment-friendly packaging for tampons, using a sustainable material such as organic cotton is preferable. The tampon pledget 10 may optionally further comprise a thermoplastic material such as polyethylene (PE), polyester or polypropylene (PP), or bicomponent fibers or a combination thereof. Such thermoplastic materials can improve the process for example. The thermoplastic material can also comprise soft carded thermobonded nonwoven material with a smooth surface to aid in the application of the hygienic product. When the tampon comprises a combination of an absorbent material and a thermoplastic material, the absorbent material is rolled up or compressed with the thermoplastic material being arranged on the outer surface of the rolled or compressed tampon pledget 10 as to cover the absorbent material. Again, given the objective of the present invention, it is preferable to use a thermoplastic material selected from a bioplastic derived from biomass such as bio-polyethylene (bio-PE) namely low-density polyethylene (LDPE) or bio-polypropylene (bio-PP).

On its outer surface, the tampon pledget 10 can comprise grooves 18 to improve fluid absorption. As illustrated in FIG. 1, the tampon pledget 10 comprises a withdrawal string 20 on its rear or trailing end 16. The trailing end 16 corresponds to the longitudinal end of tampon that is opposite to the introductory end 14. The head, or introductory end, 14 has a dome like configuration. The tampon pledget 10 can present a concave apex area 21 to improve fluid acquisition or a convex apex area to ease application of the hygienic product.

As illustrated in FIG. 2, the tampon pledget 10 is enclosed in a wrapper 22 or tampon packaging. The wrapper 22 acts as a barrier ensuring that the unused tampon pledget 10 is protected against contamination prior to use.

As shown in FIG. 3, the wrapper 22 has a generally rectangular configuration with an inner surface 23 (FIG. 2) in contact with the tampon and an outer surface 25.

The wrapper 22 according to the invention comprises cellulosic fibers or in other terms, the wrapper 22 contains a sustainable material and more specifically cellulose pulp. Preferably, the wrapper 22 comprises paper material, or cellulosic fibers, coming from natural sources for example and not limited to wood (softwood or hardwood), cotton or hemp. A nonwoven fabric of cellulosic fibers, e.g. paper, is less flexible and rougher than a plastic film. To that intent, the wrapper 22 comprises a layer consisting essentially of cellulosic fibers 33, *e.g.* a paper layer, comprising a basis weight comprised between 15 gsm and 90 gsm, preferably between 20 gsm and 70 gsm, more preferably between 25 gsm and 60 gsm, even more preferably between 25 gsm and 50 gsm. More preferably, the layer of cellulosic fibers 33 of the wrapper 22 consists essentially of paper material. By using pulp such as paper or any other natural cellulose fibers corresponding to an environment friendly material, the environmental impact of the packaging is lowered. By comprising a basis weight comprised between 15 gsm and 90 gsm, the wrapper 22 retains the functionalities of the plastic film conventionally used. The basis weight is sufficiently high to ensure that the wrapper 22 is robust enough to contain a tampon pledget 10 and not tear easily. The basis weight is sufficiently low to ensure that the wrapper 22 is flexible enough, namely for the packaging steps when enclosing the tampon within the wrapper 22. Another parameter that may be considered for the layer of cellulosic fibers 33 is its thickness, specifically, the layer of cellulosic fibers 33 has a thickness between 30 µm and 200 µm. Again, this thickness is sufficiently high to ensure that the wrapper 22 is robust enough to contain a tampon pledget 10 and not tear easily. This thickness is sufficiently low enough to ensure that the wrapper 22 is flexibleenough. Thickness of a layer can be determine using any standard technics such as measuring the wrapper or paper layer with a caliper preferably a micrometer caliper gauge. Alternatively, thickness of the layer of cellulosic fibers is preferably measured according to the ISO 534 standard or any standard method.

Depending on the dimensions of the cellulosic fibers, the layer consisting essentially of cellulosic fibers 33 can exhibit different behaviours. For example, using cellulosic fibers of higher fiber length improves the tear resistance whereas using cellulosic fibers of lesser fiber length improves the print quality and the surface smoothness of the wrapper 22. The layer consisting essentially of cellulosic fibers 33 preferably comprises cellulosic fibers having an average fiber length comprised between 0,5 and 10 mm, preferably between 0,7 and 5 mm, more preferably between 1 and 3 mm. The cellulosic fibers preferably have an average fiber diameter comprised between 0,5 and 50 µm, preferably between 5 and 30 µm. The layer consisting essentially of cellulosic fibers 33 33 has preferably a basis weight comprised between 15 gsm and 90 gsm.

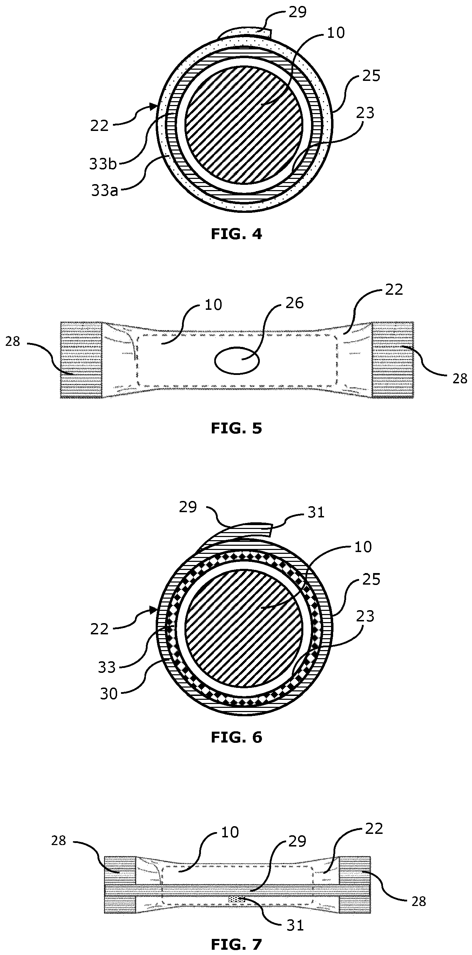

According to an embodiment (FIG.4), the layer consisting essentially of cellulosic fibers 33 comprises a first sub-layer 33a consisting essentially of cellulosic fibers and a second sub-layer 33b consisting essentially of cellulosic fibers, *e.g*. two paper layers superimposed. In other terms, the layer consisting essentially of cellulosic fibers 33 comprises a first and a second stratum 33a,33b each consisting essentially of cellulosic fibers. The first sub-layer 33a can be arranged outward radially thereby corresponding to the outer surface 25 of the wrapper. The second sub-layer 33b can be arranged between the first sub-layer 33a and the tampon pledget 10. In this embodiment, it is preferable that the first sub-layer 33a have cellulosic fibers of different physical properties that the cellulosic fibers of the second sub-layer. For example the first sub-layer 33a can comprise cellulosic fibers with an average fiber length comprised between 0,5 mm and 3 mm, whereas the second sub-layer 33b comprises cellulosic fibers with an average fiber length comprised between 2 mm and 10 mm. In other words, the second sub-layer 33b has an average fiber length that is greater than the average fiber length of the first sub-layer 33a. This way the second sub-layer 33b ensures that the wrapper 22 exhibits a satisfying tear resistance whereas the first sub-layer 33a ensures a smooth surface with printing options on the wrapper 22.

The layer consisting essentially of cellulosic fibers 33, whether comprising one layer or two sub-layers 33a,33b or more, can further comprise additives such as softeners, fillers and/or pigments, the latter to colour the paper. For example the layer consisting essentially of cellulosic fibers 33, whether comprising one layer or two sub-layers 33a,33b or more, may comprise regenerated cellulosic fibers such as rayon, viscose or acetate to increase the stiffness of the wrapper. The wrapper must be sufficiently robust to withstand processing machines and not be teared. For example if a wrapper 22 comprising one or more layers consisting essentially of cellulosic fibers 33 with a basis weight of 20 gsm, it may be preferable to add some additives such as a stiffening agent to improve resistance of the wrapper 22.

Given that cellulosic fibers, in particular paper, is a material that can easily be printed on, the wrapper 22 according to the invention can be provided with graphic elements 26 (FIG. 5) such as a print marks, letters, a logo or a drawing or a combination thereof. The graphic element 26 can be printed, by common techniques such as flexographic printing, gravure printing or inkjet printing, onto the outer surface 25 and/or inner surface 23 of the wrapper 22, in particular onto the layer consisting essentially of cellulosic fibers 33.

Given that cellulosic fibers, in particular paper, is a material that is not hydrophobic and can let moisture pass through, according to an embodiment, the wrapper 22 further comprises a layer of hydrophobic material 30 such as a thin coat of gelatine and/or collagen, a polymeric film, a plastic film, or a blister varnish. Given the objective of the present invention, providing an environment friendly wrapper, it is preferable to use a hydrophobic material that is sustainable such as beeswax, candelilla wax, botulin (lup-20(29)-ene-3beta,28-diol) that all display both hydrophobic and antibacterial properties. According to another embodiment, the layer of hydrophobic material 30 comprises a peelable layer that can be separated, in particular that can be manually or mechanically separated, e.g. peeled off, from the layer(s) consisting essentially of cellulosic fibers 33. The peelable layer can be easily separated from the layer consisting essentially of cellulosic fibers 33 for example by pulling each layer in substantially opposite directions. The paper layer and the peelable layer can be recycled or discarded separately. The peelable layer can comprise a material selected from, and not limited to, polypropylene (PP), polyethylene (PE), Polyethylene terephthalate (PET), cellophane, polyvinyl chloride (PVC), polyvinylidene chloride (PVDC) and/or a combination thereof. With such a coating, or peelable layer, the tampon pledget 10 is better protected from contamination and moisture. By adding such a coating or peelable layer, it is also possible to reduce the basis weight of the layer(s) consisting essentially of cellulosic fibers 33. The layer of hydrophobic material 30 can be arranged on the inner surface or outer surface of the layer(s) consisting essentially of cellulosic fibers 33. Preferably, the layer of hydrophobic material 30 is arranged on the inner surface of the layer(s) consisting essentially of cellulosic fibers 33 as illustrated on FIG. 6, in other words, the layer of hydrophobic material 30 defines the inner surface 23 of the wrapper 22 and the layer(s) essentially consisting of cellulosic fibers 33 defines the outer surface 25 of the wrapper 22. According to an embodiment, when there are two layers consisting essentially of cellulosic fibers 33a,33b, the layer of hydrophobic material 30 can be arranged in-between the two paper layers 33a,33b.

The wrapper 22 preferably comprises a layer of sealing agent 34 and/or undergoes a sealing treatment to obtain sealed portion 28. The sealing treatment may be carried out using any suitable means known without any particular limitation. For example, the sealing treatment can be carried out either by applying, or coating, a sealing agent on an area of the inner surface 23 of the wrapper 22, folding the wrapper 22 and absorbent article 10 and then applying temperature and/or pressure to this sealing agent to achieve the sealing. For example and not limited to, the sealing treatment can be carried out by thermo-sealing (applying heat to a heat-sealable material), ultrasonic sealing (applying ultrasounds, thus heat, to a heat-sealable material), gluing (applying an adhesive material, preferably weak such as a non-structural adhesive, and then applying pressure).

For these embodiments comprising a sealing treatment as described above, *i.e.* implying the use of a sealing agent such as an adhesive or a heat-sealable material, it is preferable that the layer of sealing agent 34 comprises a non-petroleum derived material as to lower the environmental impact of the absorbent article. The layer of sealing agent 34 comprises for example and not limited to water-based blister varnish, water-based blister lacquer, polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL). The layer of sealing agent 34 can also comprise a polymeric material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The sealing agent preferably comes from a sustainable source, or bio-based or bioplastic, and is biodegradable and/or compostable. The layer of sealing agent 34 has a basis weight comprised between 0.5 gsm and 15 gsm, preferably between 1 gsm and 10 gsm, more preferably between 2 gsm and 5 gsm.

The finished absorbent article, *i.e.* once the tampon is wrapped, comprises two sealed portions 28 arranged at both longitudinal end of the wrapper 22 and tampon pledget 10 as illustrated in FIG. 5. As illustrated in FIG. 7, the final product also comprises a side seam 29 extending in the longitudinal direction from the first sealed portion 28 to the second sealed portion 28. The side seam 29 may comprise one or more grasping elements 31, or gripping element, to ease the opening of the wrapper 22. The one or more grasping elements 31 are for example, and not limited to, a protruding tab in said side seam 29 as illustrated in FIG. 7, an uncoated portion that is free of sealing agent thereby projecting from the side seam 29 of the wrapper 22 as illustrated in FIG. 6 or a notch in said side seam 29 or a combination thereof. The side seam 29 itself can be considered as a grasping element 31.

Alternatively, the wrapper 22 may comprise a tearing element such as pre-cut marks or perforations to ease the opening of the wrapper 22. Of course, the wrapper may comprise both grasping element 31 and tearing element.

The one or more layer(s) consisting essentially of cellulosic fibers 33, the layer of sealing agent 34 and optionally the layer of hydrophobic material 30 form a laminate.

Fig. 3 illustrates a top view, meaning facing the inner surface 23, of the wrapper 22 in an unfolded or unrolled configuration, or in other terms laid flat, with a tampon pledget 10 placed upon it, on the inner surface 23 of the wrapper, for visualisation reasons. The wrapper 22 has a generally rectangular configuration comprising a front end 38, a back end 40, or rear end, arranged at the opposite side of the front end 38 and a first and second side ends 42, 46 arranged in-between. The front end 38, back end 40 and side ends 42,46 each having an edge, the side end 42,46 edges linking the front end 38 edge to the back end 40 edge. The front end 38 is arranged adjacent to the introductory end 14 of the tampon pledget 10 whereas the back end 40 is arranged adjacent to the trailing end 16 of the tampon pledget 10.

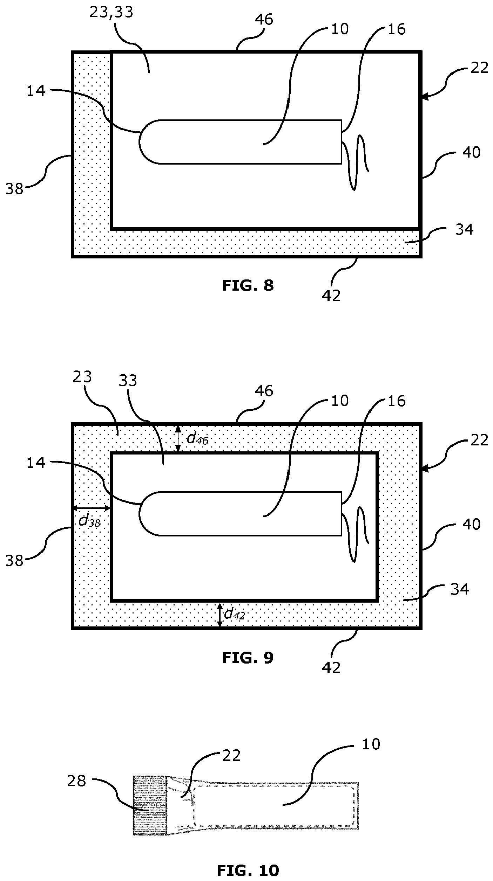

The wrapper 22 of the present invention preferably has sealing properties where an area of the inner surface 23 and/or outer surface 25 of the wrapper 22 is subjected to a sealing treatment. For example the peripheral region of the inner surface 23 of the wrapper 22 can be subjected to a sealing treatment. According to embodiments such as the one illustrated in FIG. 3, Fig. 8 or Fig. 9, a minority of the area of the inner surface 23 is subjected to sealing treatment, more precisely, only the area comprising the peripheral edge portion of the inner surface 23 is subjected to a sealing treatment. In other words, the wrapper 22 preferably comprises a layer of sealing agent 34, that layer, or coat, or coating, being arranged preferably on the inner surface 23 of the wrapper 22 at least along the front and/or back ends 38,40 edges and the first and/or second side ends 42,46 edges as to enable the sealed portions 28 and side seam 29, the rest of the surface being the layer consisting essentially of cellulosic layer 33 (FIG. 8, FIG. 9) and/or the layer of hydrophobic material 30 (FIG. 3).

The layer of sealing agent 34 may be arranged along a portion of, or at least partially along one side end edges, preferably along the entire length of the wrapper 22 along said side ends 42 edges (FIG. 3) thereby defining a side seam 29 extending over the entire longitudinal length of the wrapper as illustrated in FIG. 7. Additionally, the layer of sealing agent 34 may be arranged along a portion of, or at least partially along both side end 42,46 edges, preferably along the entire length of the wrapper 22 along both side end 42,46 edges (FIG. 9) thereby defining a side seam 29 extending over the entire longitudinal length of the wrapper as illustrated in FIG. 7. The side seam 29 can be formed by folding the coated side end 42 edge over the uncoated side end 46 edge (Fig.3) and applying heat and/or pressure onto the sealing agent. The side seam 29 can be formed by associating the two coated side end 42,46 edges, specifically associating the two surfaces of the side end 42,46 edges where the sealing agent is arranged, against one another, applying heat and/or pressure and folding the joined edges against uncoated portion of the wrapper 22.

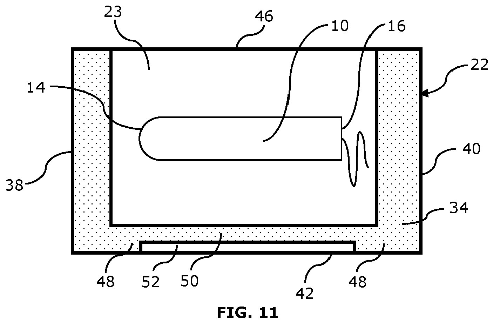

According to the embodiment illustrated in FIG. 11, the layer of sealing agent 34 is arranged along one or more portions 48 of side end 42 edge and along the entire length of the wrapper 22 at a region 50 adjacent to side end 42 edge but at a distance of said side end 42 edge thereby defining uncoated portions 52 at the side end 42 edge. In other terms, the layer of sealing agent 34 arranged at the side end 42 edge comprises a notch, *i.e.* the uncoated portion. We can also say that there is a gap, *i.e.* free of sealing agent, between the side end 42 edge and the coated region 50. The coated region 50 and the portion 48 define the side seam 29 extending over the entire longitudinal length of the wrapper as illustrated in FIG. 7 and the uncoated portion 52 defines a grasping element 31, in this case a tab, or tongue that can be gripped. Indeed, when folding the side end 42 edge over the over side end 46 edge, applying heat and/or pressure will fix the coated portions and region 48,50 onto the other side end whilst the uncoated portion 52 remains unattached.

The layer of sealing agent 34 may be arranged along a portion of, or at least partially along both front and back end edges, preferably along the entire width of the wrapper 22 along the front end 38 edge and along the back end edge 38 (FIG. 3) thereby defining two sealed portions 28 as illustrated in FIG. 5 arranged at each longitudinal ends of the tampon pledget 10. Alternatively, the layer of sealing agent 34 may be arranged along a portion of, or at least partially along, the front end 38 edge or the back end 40 edge exclusively, preferably along the entire width of the wrapper 22 along either the front or back end 38,40 edges (FIG. 9) thereby defining one sealed portion 28 as illustrated in FIG. 10 arranged at one longitudinal end of the tampon pledget 10. The sealed portion(s) 28 can be formed by folding the first side end 42 edge over the second side end 46 edge and applying heat and/or pressure onto the sealing material to the front end 38 and back end 40. In other words, the lateral extremities of the front end 38 are bonded and the lateral extremities of the back end 40 are bonded.

The layer of sealing agent 34 can be arranged on the entire periphery of the wrapper 22 as illustrated in FIG 9 to have an improved sealing.

The coat of sealing agent 34 can be applied along an edge of the wrapper within a certain distance d from the edge (as illustrated in FIG. 3, 8 and 9), d is comprised between 2 mm and 20 mm, preferably between 5 mm and 10 mm. Such dimensions enable a good sealing of the packaging and ensure that the tampon pledget 10 is protected against contamination and maintaining a low environment impact. The distance *dₓ* refers to the distance on which sealing agent 34 is applied on the paper layer 33 relative to the front and/or back and/or side ends 38, 40, 42, 46 edges. The distance *d₃₈* of sealing agent 34 at the front end 38 edge preferably have the same value, e.g. between 5 mm and 10 mm, as the distance *d₄₀* of sealing agent at the back end 40 edge. The distance *d₄₂,d₄₆* of sealing agent 34 at the side end 42,46 edges preferably have the same dimensions. Alternatively, the distance *d₄₂* of sealing agent 34 at one side end 42 edge can be greater than the distance *d₄₆* of sealing agent 34 at the opposite side end 46 edge. Preferably the distance *d₃₈,d₄₀* of sealing agent 34 at the front or back 38,40 edge is greater than the distance *d₄₂,d₄₆* of sealing agent 34 at the side end 42,46 edges.

Should the wrapper 22 comprise a layer of hydrophobic material 30 meaning the wrapper 22 comprises one or more layers consisting essentially of cellulosic fibers 33 on which is applied a coating of sealing agent 34 and a coating of hydrophobic material 30, preferably the coating of sealing agent 34 and the coating of hydrophobic material 30 do not overlap one another. The layer of sealing agent 34 is arranged on the periphery of the wrapper 22, along some of the edges whereas the layer of hydrophobic material 30 is arranged at a central region of the inner surface 23 of the wrapper 22 as illustrated in FIG. 3. Another way to say is that the sealing agent is arranged at least partially around the hydrophobic material, or that the hydrophobic material is at least partially surrounded by the sealing agent. The layer of sealing agent 34 can be arranged on one side of the wrapper 22 and the layer of hydrophobic material 30 can be arranged on the other side of the wrapper.

It is supposed that the present invention is not restricted to any form of realization described previously and that some modifications can be added to the presented example of fabrication without reappraisal of the appended claims.

## Claims

1. Wrapper (22) for a tampon (10), said wrapper comprising a layer consisting essentially of cellulosic fibers (33) and a layer of sealing agent (34), wherein the layer consisting essentially of cellulosic fibers (33) comprises a basis weight comprised between 15 gsm and 90 gsm.

2. Wrapper (22) according to claim 1, wherein the layer consisting essentially of cellulosic fibers (33) consists essentially of paper material.

3. Wrapper (22) according to any of the preceding claims, wherein the wrapper (22) further comprises a layer of hydrophobic material (30).

4. Wrapper (22) according to claim 3, wherein the layer of hydrophobic material (30) comprises a peelable layer that can be separated from the layer consisting essentially of cellulosic fibers (33).

5. Wrapper (22) according to any of the preceding claims, wherein the layer consisting essentially of cellulosic fibers (33) comprises a thickness comprised between 30 µm to 200 µm.

6. Wrapper (22) according to any of the preceding claims, wherein the layer consisting essentially of cellulosic fibers (33) comprises cellulosic fibers with an average fiber diameter comprised between 0,5 and 50 µm.

7. Wrapper (22) according to any of the preceding claims, wherein the layer consisting essentially of cellulosic fibers (33) comprises a first sub-layer of cellulosic fibers (33a) and a second layer of cellulosic fibers (33b), the cellulosic fibers in the second sub-layer (33b) having a greater basis weight than the first sub-layer (33a).

8. Wrapper (22) according to any of the preceding claims, wherein the layer of sealing agent (34) has a basis weight comprised between 0.5 gsm and 15 gsm, preferably between 1 gsm and 10 gsm, more preferably between 2 gsm and 5 gsm.

9. Wrapper (22) according to any of the preceding claims, wherein the sealing agent is selected from water-based blister varnish, polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid.

10. Wrapper (22) according to any of the preceding claims, wherein the wrapper (22) comprises a rectangular configuration, the layer of sealing agent (34) being arranged on the periphery of the wrapper (22) along at least two contiguous edges (38,40,42,46).

11. Wrapper (22) according to any of the preceding claims, wherein the layer consisting essentially of cellulosic fibers (33) comprises regenerated cellulosic fibers selected from viscose, acetate and/or rayon.

12. Wrapper according to any of the preceding claims, wherein said wrapper comprises a grasping element (31).

13. Wrapper (22) according to claim 10, wherein the wrapper (22) comprises uncoated portions (52) arranged between an edge (42,46) of the wrapper (22) and a portion of the wrapper (22) coated by a layer of sealing agent (34).

14. Wrapper (22) according to any of the preceding claims, wherein said wrapper (22) comprises at least one graphic element (26).

15. Assembly comprising a wrapper (22) according to any of the claims 1 to 14 and a tampon pledget (10).
